# EUROPEAN PATENT APPLICATION

(11) **EP 4 242 293 A1**
(43) Date of publication of application: **13.09.2023**
(21) Application number: 21900636.8
(22) Date of filing: 01.12.2021
(51) Int. Cl.: C12M 3/00, C12N 5/071

(54) **CULTURE VESSEL, AND METHOD OF USING CULTURE VESSEL**

(30) Priority: 02.12.2020 JP 2020200057
(71) Applicant: Ushio Denki Kabushiki Kaisha, Tokyo 100-8150 (JP)
(72) Inventor: YAMANAKA, Makoto, Tokyo 100-8150 (JP)
(74) Representative: Tomerius, Isabel
(86) International application number: PCT/JP2021/044100
(87) International publication number: WO 2022/118885

(57) **Abstract**

Provided are a culture vessel that makes it possible to simulate cellular response in a manner similar to in vivo and collect fluid secreted from cells at a high concentration and a method of using such a culture vessel. A culture vessel 1 has a culture space 3 therein and is capable of culturing cells in the culture space 3, the culture vessel 1 including a main flow path 21 that extends within a plane, and a secondary flow path 11 that is adjacent to the main flow path 21 in a direction perpendicular to the plane, extends in a direction intersecting the main flow path 21 when viewed from a direction perpendicular to the plane, and partially overlaps the main flow path 21, wherein the secondary flow path 11 has a groove 11a that is open to and communicates with the main flow path 21 in an area 12 where the secondary flow path 11 overlaps the main flow path 21, and the culture space 3 is a space within the main flow path 21, the space being opposed to a contact surface 3a where the secondary flow path 11 is in contact with the main flow path 21 in the area 12 where the secondary flow path 11 overlaps the main flow path 21.

## Description

### TECHNICAL FIELD

The present invention relates to a culture vessel and a method of using the culture vessel.

### BACKGROUND ART

In the liver, drugs or other compounds are absorbed into and metabolized by hepatocytes. Hepatic metabolites are excreted into bile canaliculi, reach the gallbladder, and are discharged from the body as feces through the intestines.

As a conventional method for collecting fluid secreted from hepatocytes, a cell culture method using a cell culture insert is known.

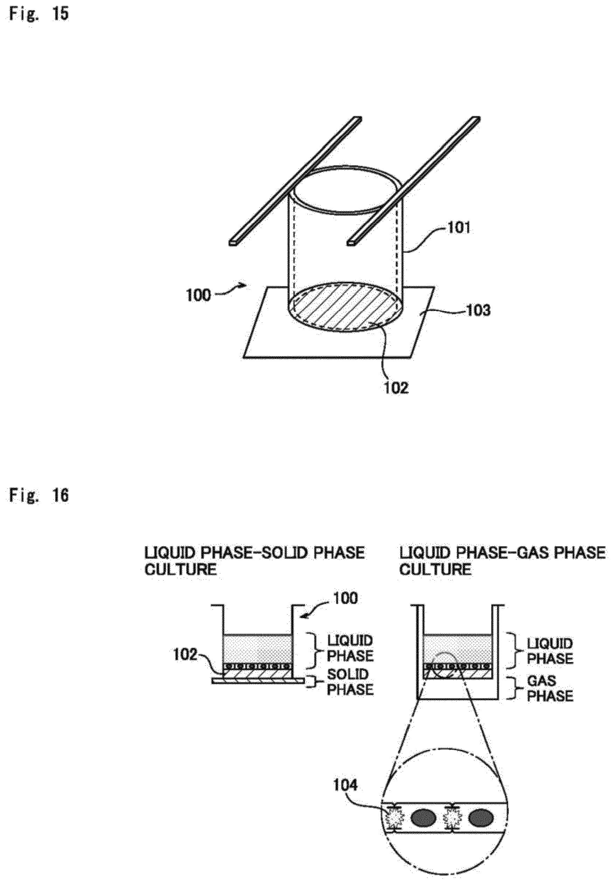

Fig. 15 shows an example of a conventional transportable culture vessel 100 having a membrane that can pass a physiologically active substance. A frame 101 has a space for holding cells inside a cylindrical tube made of acrylic or polystyrene, and a membrane 102 that can pass a physiologically active substance is fixed to one of the ends of the frame 101 by bonding with an adhesive. The membrane 102 has a PET release film 103 adhering thereto.

The membrane 102 that can pass a physiologically active substance is obtained by gelatinization of an artificial material made of a naturally occurring or synthetic polymer by introduction of cross-links. As an extracellular matrix (ECM) component to be gelatinized, for example, collagen, hyaluronic acid, gelatin, agar, agarose, or the like can be used.

As shown in Fig. 16, the culture vessel 100 having the membrane 102 that can pass a physiologically active substance can be transported while holding a culture such as cells therein to perform culture in different environments such as "liquid phase-membrane-solid phase", "liquid phase-membrane-gas phase", and "liquid phase-membrane-liquid phase".

Particularly, cell function assay can be performed using a transdermal absorption model or an intestinal absorption model having epithelial-mesenchymal interaction and obtained by respectively culturing epithelial cells and mesenchyme cells on one surface and the other surface of the membrane 102 that can pass a physiologically active substance, or an angiogenic model or a cancer invasion model obtained by respectively culturing vascular endothelial cells and cancer cells on one surface and the other surface of the membrane 102 that can pass a physiologically active substance.

Such transportability makes it possible to construct a hepatocyte culture device that promotes accumulation and excretion of hepatic metabolites in and into a bile canaliculus-like structure 104.

### PRIOR ART DOCUMENT

### PATENT DOCUMENT

Patent Document 1: JP-A-2020-28305

### SUMMARY OF THE INVENTION

### PROBLEMS TO BE SOLVED BY THE INVENTION

However, such a conventional culture vessel is large in volume, and therefore it is not possible to simulate cellular response resulting from interaction between cells through a physiologically active substance in a manner similar to in vivo.

Further, fluid secreted from cells is greatly diluted with a liquid in the culture device, and therefore it is difficult to collect fluid secreted from cells at a high concentration.

In light of the above problems, it is an object of the present invention to provide a culture vessel that makes it possible to simulate cellular response in a manner similar to in vivo and collect fluid secreted from cells at a high concentration, and a method of using such a culture vessel.

### MEANS FOR SOLVING THE PROBLEMS

The present invention is directed to a culture vessel that has a culture space therein and is capable of culturing cells in the culture space, the culture vessel including:
a main flow path that extends within a plane; and
a secondary flow path that is adjacent to the main flow path in a direction perpendicular to the plane, extends in a direction intersecting the main flow path when viewed from a direction perpendicular to the plane, and partially overlaps the main flow path, wherein
the secondary flow path has a groove that is open to and communicates with the main flow path in an area where the secondary flow path overlaps the main flow path, and
the culture space is a space within the main flow path, the space being opposed to a contact surface where the secondary flow path is in contact with the main flow path in the area where the secondary flow path overlaps the main flow path.

Since this culture vessel includes the secondary flow path partially overlapping the main flow path and has the culture space within the main flow path in an area where the secondary flow path overlaps the main flow path, cells can be cultured in the culture space and fluid secreted from the cells can be collected through the groove of the secondary flow path, which makes it possible to simulate (reproduce, functionally reproduce, mimic, functionally mimic, model) cellular response in a manner similar to in vivo and collect fluid secreted from cells at a high concentration.

The present invention is also directed to a method of using the culture vessel, the method including:
a first step of introducing cells into the main flow path;
a second step of fixing the cells to the culture space;
a third step of culturing the cells; and
a fourth step of collecting fluid excreted from the cultured cells into the groove.

The method of using the culture vessel makes it possible to simulate (reproduce, functionally reproduce, mimic, functionally mimic, or model) cellular response in a manner similar to in vivo and collect fluid secreted from cells at a high concentration.

### BRIEF DESCRIPTION OF THE DRAWINGS

Fig. 1 is a plan view of a culture vessel according to the present embodiment.
Fig, 2A is a sectional view of the culture vessel shown in Fig. 1 taken along line A-A.
Fig, 2B is a sectional view of the culture vessel shown in Fig. 1 taken along line B-B.
Fig. 2C is a sectional view of the culture vessel shown in Fig. 1 taken along line C-C.
Fig. 2D is a sectional view of the culture vessel shown in Fig. 1 taken along line D-D.
Fig. 2E is a sectional view of the culture vessel shown in Fig. 1 taken along line E-E.
Fig. 3 is a perspective view around a third port.
Fig. 4A is a perspective view of a first substrate.
Fig. 4B is a perspective view of a second substrate.
Fig. 5 is a schematic diagram for describing usage example 1 of the culture vessel.
Fig. 6 is a schematic diagram for describing usage example 2 of the culture vessel.
Fig. 7 is a schematic diagram for describing usage example 3 of the culture vessel.
Fig. 8 is a schematic diagram for describing usage example 4 of the culture vessel.
Fig. 9A is a sectional view for describing usage example 5 of the culture vessel.
Fig. 9B shows sectional views for describing usage example 5 of the culture vessel.
Fig. 10 is a schematic diagram for describing usage example 6 of the culture vessel.
Fig. 11A is a schematic diagram for describing usage example 6 of the culture vessel.
Fig. 11B is a schematic diagram for describing usage example 6 of the culture vessel.
Fig. 11C is a schematic diagram for describing usage example 6 of the culture vessel.
Fig. 11D is a schematic diagram for describing usage example 6 of the culture vessel.
Fig. 12A is a plan view of a culture vessel according to another embodiment.
Fig. 12B is a plan view of a culture vessel according to another embodiment.
Fig. 12C is a plan view of a culture vessel according to another embodiment.
Fig. 13A is a plan view of a culture vessel according to another embodiment.
Fig. 13B is a plan view of a plate including a plurality of culture vessels.
Fig. 13C is a plan view of a culture vessel according to another embodiment.
Fig. 14A is a plan view of a culture vessel according to another embodiment.
Fig. 14B is a plan view of a culture vessel according to another embodiment.
Fig. 14C shows a plan view and a sectional view of a culture vessel according to another embodiment.
Fig. 14D shows a plan view and sectional views of a culture vessel according to another embodiment.
Fig. 15 is a perspective view showing an example of a conventional culture vessel.
Fig. 16 is a sectional view showing a usage example of the conventional culture vessel.

### MODE FOR CARRYING OUT THE INVENTION

A culture vessel according to the present invention will be described with reference to the drawings. It should be noted that the drawings disclosed herein merely show schematic illustrations. Namely, the dimensional ratios on the drawings do not necessarily reflect the actual dimensional ratios, and the dimensional ratios are not necessarily the same between the drawings.

### [Structure]

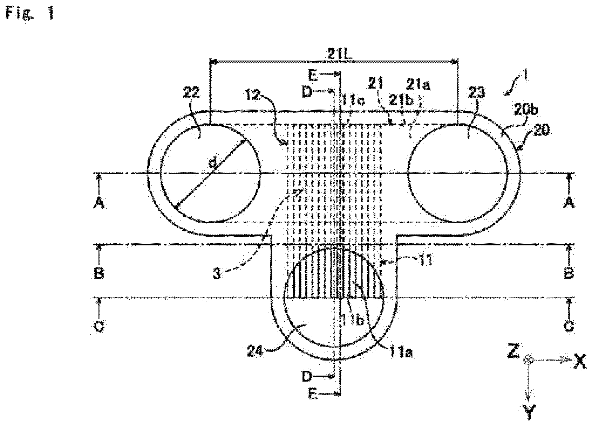

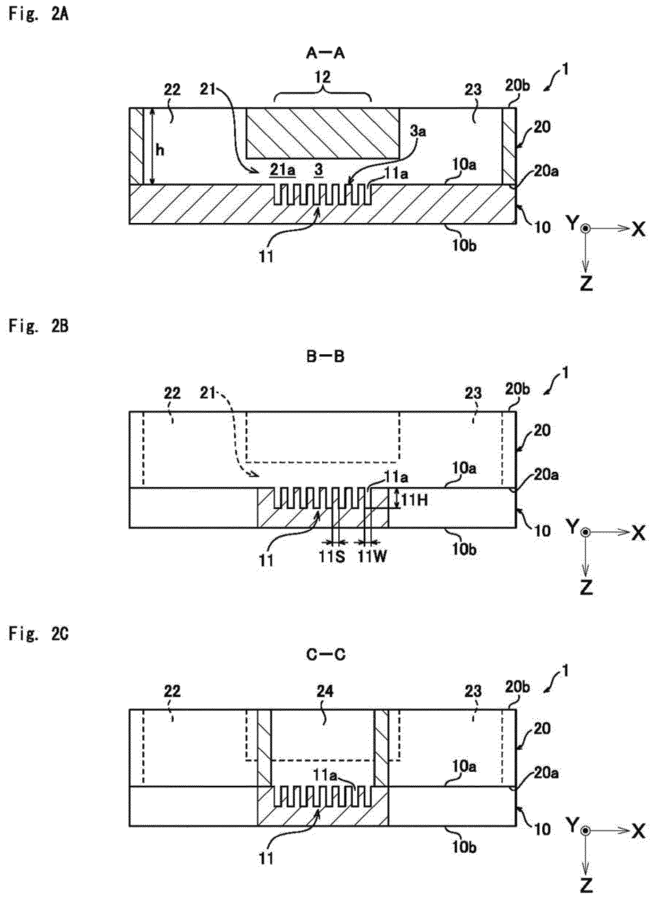

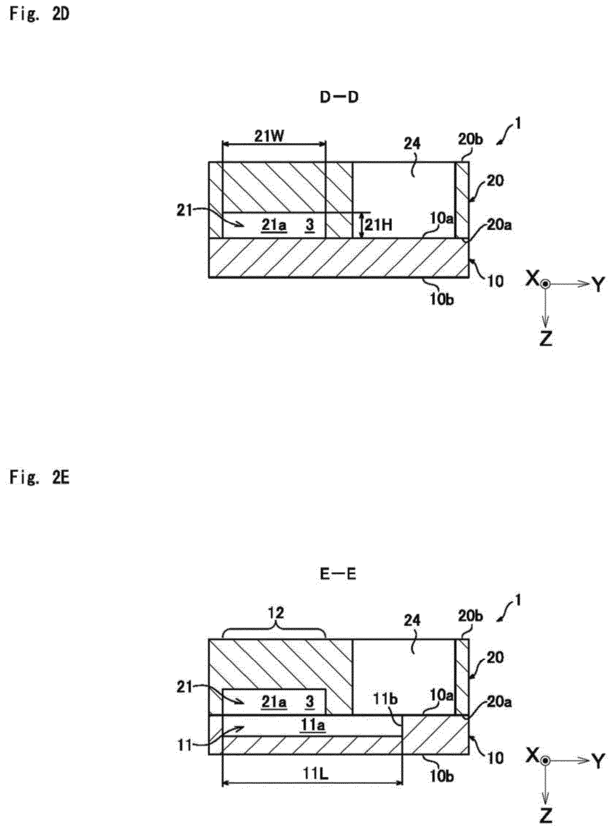

Fig. 1 is a plan view of a culture vessel 1 according to the present embodiment. Fig, 2A is a sectional view of the culture vessel 1 shown in Fig. 1 taken along line A-A. Fig, 2B is a sectional view of the culture vessel 1 shown in Fig. 1 taken along line B-B. Fig. 2C is a sectional view of the culture vessel 1 shown in Fig. 1 taken along line C-C. Fig. 2D is a sectional view of the culture vessel 1 shown in Fig. 1 taken along line D-D. Fig. 2E is a sectional view of the culture vessel 1 shown in Fig. 1 taken along line E-E.

The culture vessel 1 is formed by stacking a second substrate 20 on a first substrate 10 so that one principal surface 20a of the second substrate 20 is partially in contact with one principal surface 10a of the first substrate 10 and bonding them together. The principal surface refers to one of surfaces constituting the substrate 10 or 20 and having a much larger area than other surfaces. The substrate 10 or 20 has two principal surfaces, and these two principal surfaces are opposed to each other.

The principal surface 10a of the first substrate 10 has a plurality of grooves 11a (which will be described later in detail). Further, the principal surface 20a of the second substrate 20 has a recess 21a (which will be described later in detail). The other principal surface 20b of the second substrate 20 is located on the opposite side from the first substrate 10 and has first to third ports 22 to 24 (which will be described later).

In the following description, an XYZ coordinate system is appropriately referenced in which, in a state where the first substrate 10 and the second substrate 20 are bonded together, a plane parallel to the principal surfaces 10a and 10b of the first substrate 10 and the principal surfaces 20a and 20b of the second substrate 20 is defined as an XY plane and a direction orthogonal to the XY plane is defined as a Z direction.

When it is necessary to make a distinction between positive or negative to express a direction herein, the direction is described with a positive or negative sign, such as "+X direction" or "-X direction". When it is not necessary to make a distinction between positive or negative to express a direction, the direction is simply described as "X direction". Namely, when the direction is simply described as "X direction" herein, both "+X direction" and "-X direction" are included. The same applies to the Y direction and the Z direction. It should be noted that the culture vessel 1 is usually used so that the Z direction corresponds to a vertical direction, and the -Z direction corresponds to an upward direction.

The first substrate 10 and the second substrate 20 are the same in the shape of principal surfaces. In the culture vessel 1 of the present embodiment, the first substrate 10 and the second substrate 20 have an almost T-shape when viewed from the Z direction. The thickness of the second substrate 20 is larger than that of the first substrate 10. The thickness of the first substrate 10 is, for example, 0.1 to 1 mm, and the thickness of the second substrate 20 is, for example, 3 to 10 mm.

The principal surface 10a of the first substrate 10 includes grooves 11a. The grooves 11a are formed in the principal surface 10a so as to extend in the Y direction. The grooves 11a are formed to be arranged side by side in the X direction. When the first substrate 10 and the second substrate 20 are bonded together, the grooves 11a function as a secondary flow path 11 sandwiched between both of the substrates 10 and 20. The number of the grooves 11a is, for example, 10 to 1000. In Figs. 1 to 3, only 8 grooves 11a are shown for the purpose of illustration.

Each of the grooves 11a has a slit shape that extends in the Y direction so as to have constant width and depth. Each of the grooves 11a has a rectangular sectional shape. However, the sectional shape of each of the grooves 11a is not limited to a rectangular shape, and may be a trapezoidal or semi-elliptical shape such that the width decreases toward the groove bottom.

Each of the grooves 11a has a width 11W (see Fig. 2B) of, for example, 5 to 100 µm in the X direction. In the present embodiment, the width 11W is 50 µm. The width 11W of each of the grooves 11a is a width at an opening that is open to the principal surface 10a. A space 11S (see Fig. 2B) between the adjacent grooves 11a is, for example, one to two times the width 11W, and is 50 µm in the present embodiment. Each of the grooves 11a has a depth 11H (see Fig. 2B) of, for example, 50 to 150 µm. In the present embodiment, the depth 11H is 50 µm. Each of the grooves 11a has a length 11L (see Fig. 2E) of, for example, 4 to 20 mm. In the present embodiment, the length 11L is 6 mm.

The principal surface 20a of the second substrate 20 includes a recess 21a. The recess 21a is formed in the principal surface 20a so as to extend in the X direction. When the first substrate 10 and the second substrate 20 are bonded together, the recess 21a functions as a hollow main flow path 21 sandwiched between both of the substrates 10 and 20.

The recess 21a has a slit shape that extends in the X direction so as to have constant width and depth. The recess 21a has a rectangular sectional shape.

The recess 21a has a width 21W (see Fig. 2D) of, for example, 1 to 10 mm in the Y direction. The recess 21a has a depth 21H (see Fig. 2D) of, for example, 0.1 to 5 mm. The recess 21a has a length 21L (see Fig. 1) of, for example, 4 to 40 mm.

By forming the grooves 11a in the principal surface 10a of the first substrate 10 and forming the recess 21a in the principal surface 20a of the second substrate 20, the main flow path 21 is located within a first XY plane and the secondary flow path 11 is located within a second XY plane different from the first XY plane when the first substrate 10 and the second substrate 20 are bonded together. That is, the secondary flow path 11 is adjacent to the main flow path 21 in the Z direction.

The first port 22 is connected to the -X direction-side end of the recess 21a, and is formed to extend from the principal surface 20a toward the principal surface 20b of the second substrate 20 and to penetrate the second substrate 20. The second port 23 is connected to the +X direction-side end of the recess 21a, and is formed to extend from the principal surface 20a toward the principal surface 20b of the second substrate 20 and to penetrate the second substrate 20.

The third port 24 is formed in a position independent from the recess 21a to extend from the principal surface 20a toward the principal surface 20b of the second substrate 20 and to penetrate the second substrate 20. The third port 24 is formed in a position opposed to ends 11b of the grooves 11a formed in the first substrate 10 in a state where the first substrate 10 and the second substrate 20 are bonded together. This allows the third port 24 to be connected to the ends 11b of the grooves 11a when the first substrate 10 and the second substrate 20 are bonded together.

The first port 22, the second port 23, and the third port 24 are all cylindrical hollows extending in the Z direction. In the culture vessel 1 of the present embodiment, the first port 22, the second port 23, and the third port 24 are all the same in diameter d (see Fig. 1), and the diameter d is, for example, 1 to 10 mm. It should be noted that it is not necessary for the first port 22, the second port 23, and the third port 24 to have the same diameter d. The first port 22, the second port 23, and the third port 24 are all the same in height h (see Fig. 2A), and the height h is, for example, 3 to 10 mm. The distance between the centerline of the main flow path 21 (recess 21a) and the center of the third port 24 in the Y direction is, for example, 3 to 10 mm.

The first port 22 and the second port 23 are connected through the main flow path 21. That is, the main flow path 21 can be said to have the first port 22 and the second port 23 which are connected to the main flow path 21 and extend toward the principal surface 20b.

The first port 22 and the second port 23 have at least one of the purpose of supplying a liquid to the culture vessel 1 and the purpose of discharging a liquid from the culture vessel 1. For example, the first port 22 and the second port 23 may be used as a supply port and a discharge port, respectively.

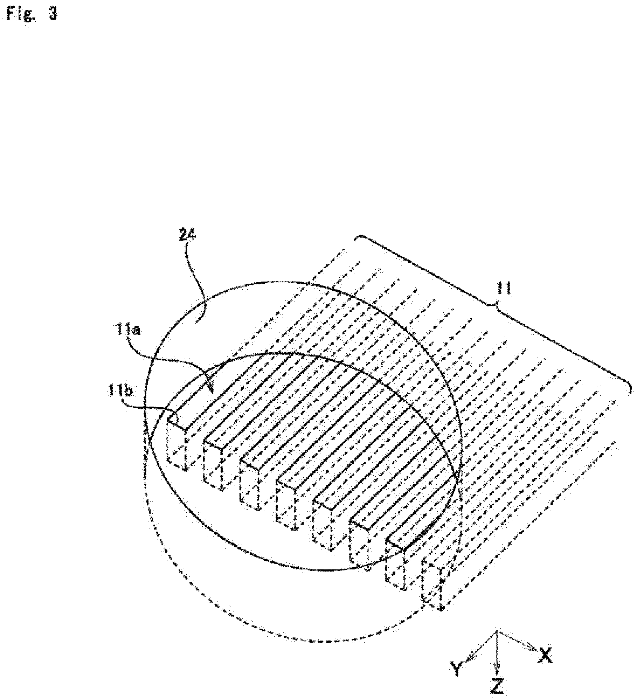

As shown in Fig. 1 and Fig. 3, in a state where the first substrate 10 and the second substrate 20 are bonded together, the grooves 11a are formed so that the ends 11b are located on a straight line extending through the center of the third port 24 in the X direction. This allows the ends 11b of the grooves 11a to communicate with the third port 24. As shown in Fig. 1, other ends 11c of the grooves 11a are located at a -Y direction-side edge 21b of the recess 21a. The grooves 11a have a constant length 11L. A constant length 11L of the grooves 11a makes flow path resistance constant.

In a state where the first substrate 10 and the second substrate 20 are bonded together, the grooves 11a extend in the Y direction intersecting the recess 21a and partially overlap the recess 21a when viewed from the Z direction. That is, when viewed from the Z direction, the secondary flow path 11 extends in the Y direction intersecting the main flow path 21 and partially overlaps the main flow path 21. Here, an area where the secondary flow path 11 and the main flow path 21 overlap each other when viewed from the Z direction is referred to as an "overlapping area 12".

The grooves 11a are opposed to the recess 21a in a state where the first substrate 10 and the second substrate 20 are bonded together. This allows the grooves 11a to be open to and communicate with the main flow path 21 in the overlapping area 12.

The culture vessel 1 has a culture space 3 therein, and cells can be cultured in the culture space 3. The culture space 3 is a space within the main flow path 21 in the overlapping area 12. The culture space 3 is opposed to a contact surface 3a (see Fig. 2A) where the secondary flow path 11 is in contact with the main flow path 21 in the overlapping area 12. Although described later in detail, cells introduced into the main flow path 21 adhere to the bottom surface of the culture space 3, specifically the contact surface 3a, more specifically the principal surface 10a of the first substrate 10 in the overlapping area 12. At this time, the cells adhere onto the secondary flow path 11 so as to cover the grooves 11a.

The third port 24 is connected to the secondary flow path 11. That is, the secondary flow path 11 can be said to have the third port 24 connected to the secondary flow path 11 and extending toward the principal surface 20b.

The third port 24 has the purpose of discharging a liquid in the grooves 11a to the outside. For example, the third port 24 may be used as a collection port to collect a liquid in the grooves 11a.

As described above, the culture vessel 1 according to the present embodiment has the culture space 3 therein, is capable of culturing cells in the culture space 3, and includes the main flow path 21 and the secondary flow path 11. The main flow path 21 extends within the XY plane. The secondary flow path 11 is adjacent to the main flow path 21 in a direction (Z direction) perpendicular to the XY plane, extends in a direction intersecting the main flow path 21 when viewed from a direction (Z direction) perpendicular to the XY plane, and partially overlaps the main flow path 21. The secondary flow path 11 has the grooves 11a that are open to and communicate with the main flow path 21 in the overlapping area 12 where the secondary flow path 11 overlaps the main flow path 21. The culture space 3 is a space within the main flow path 21, the space being opposed to the contact surface 3a where the secondary flow path 11 is in contact with the main flow path 21 in the overlapping area 12 where the secondary flow path 11 overlaps the main flow path 21.

In the culture vessel 1 according to the present embodiment, the contact surface 3a preferably has cell adhesiveness. Cell adhesiveness means the property of a surface having a chemical bond as a scaffold for cell adhesion or the property of a surface coatable with an ECM component (such as collagen, gelatin, or laminin), for example, a surface having a functional group such as a hydroxyl group or a carboxy group.

In the culture vessel 1 according to the present embodiment, it is preferred that the width 21W of the main flow path 21 is larger than a cell to be cultured and the width 11W of each of the grooves 11a is smaller than the cell to be cultured.

Such a configuration of the culture vessel 1 as described above makes it possible to simulate cellular response in a manner similar to in vivo and collect fluid secreted from cells at a high concentration, which will be described later with reference to a method of using the culture vessel 1.

### [Production method]

Hereinbelow, a method for producing the culture vessel 1 will be described in detail.

### (Substrate preparing step)

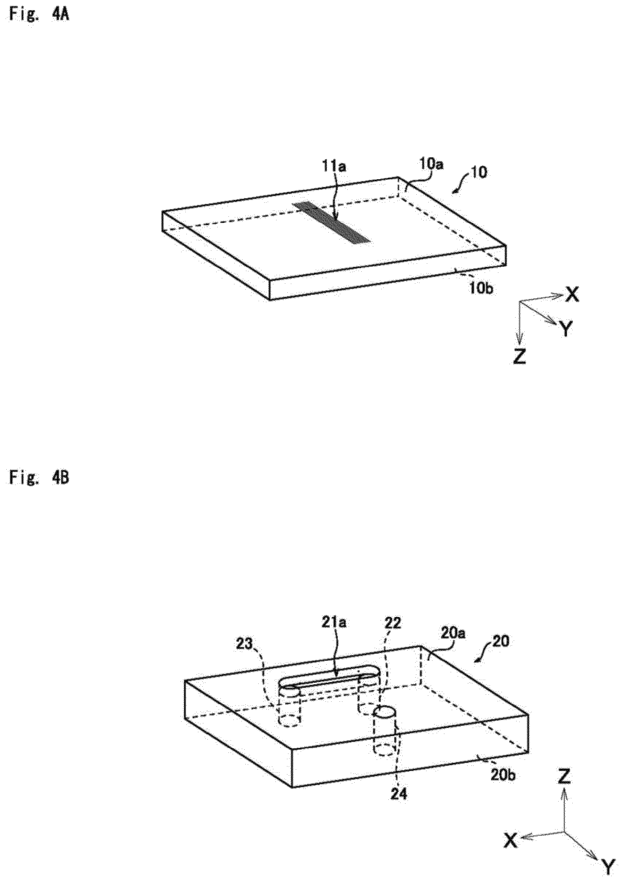

The first substrate 10 and the second substrate 20 to form the culture vessel 1 are prepared. Fig. 4A is a perspective view of the first substrate 10 before bonding when viewed from the principal surface 10a side, and Fig. 4B is a perspective view of the second substrate 20 before bonding when viewed from the principal surface 20a side.

A material used to form the substrates 10 and 20 is preferably a substantially non-porous material. The "substantially non-porous material" herein refers to a state where the apparent surface area of the substrate is approximate to the actual surface area. Examples of a material to form such a non-porous material include an inorganic material such as glass or silicon and a resin material such as polymethyl methacrylate (PMMA), polycarbonate (PC), cycloolefin copolymer (COC), cycloolefin polymer (COP), polystyrene (PS), or silicone. It should be noted that these resin materials may be used in combination of two or more of them. The material used to form the first substrate 10 and the material used to form the second substrate 20 may be different.

The shape of the substrates in the present embodiment will be described. As the first substrate 10 and the second substrate 20, substrates that are the same in the shape of principal surfaces are used. The thickness of the second substrate 20 is larger than that of the first substrate 10. However, the first substrate 10 and the second substrate 20 may be different in the shape of principal surfaces. For example, the lengthwise dimension and widthwise dimension of principal surface of the first substrate 10 may be larger than those of principal surface of the second substrate 20, or the lengthwise dimension and widthwise dimension of principal surface of the second substrate 20 may be larger than those of principal surface of the first substrate 10. The thickness of the second substrate 20 may be the same as that of the first substrate 10, or the thickness of the second substrate 20 may be smaller than that of the first substrate 10.

The principal surface 10a of the first substrate 10 has the grooves 11a. The grooves 11a are formed by, for example, a combination of a photolithographic process and an etching process, nanoimprinting, laser processing, injection molding, or milling.

The principal surface 20a of the second substrate 20 has the recess 21a. The other principal surface 20b of the second substrate 20 has openings as the first port 22, the second port 23, and the third port 24.

In order to provide openings and a recess in the second substrate 20, for example, a means such as injection molding or cutting work may be used, but an optimum means may be selected depending on the material forming the substrate. For example, as described above, when the second substrate 20 is formed using a resin material such as polymethyl methacrylate (PMMA), polycarbonate (PC), cycloolefin copolymer (COC), cycloolefin polymer (COP), polystyrene (PS), silicone, or acrylic, a recess can easily be formed by injection molding.

### (Substrate bonding step)

The principal surface 10a of the produced first substrate 10 and the principal surface 20a of the produced second substrate 20 are bonded together. A bonding method described below does not require formation of a thin film as an adhesive on the substrate and is performed in the following procedure.

First, the bonding surfaces (10a, 20a) of both of the substrates are subjected to surface activation treatment. As a method of the surface activation treatment, a method including irradiation with ultraviolet rays or a method including contact with plasma gas can be used.

The method including irradiation with ultraviolet rays is performed by, for example, irradiating the principal surface 20a of the second substrate 20 and the principal surface 10a of the first substrate 10 with vacuum ultraviolet rays having a wavelength of 200 nm or less emitted from an ultraviolet light source such as a xenon excimer lamp to emit light having a wavelength of 172 nm. As another example of the ultraviolet light source, a low-pressure mercury lamp having an emission line at 185 nm or a deuterium lamp having an emission line in a wavelength range of 120 to 200 nm can suitably be used. The illuminance of the vacuum ultraviolet rays is, for example, 10 to 500 mW/cm². An irradiation time is appropriately set depending on the type of resin used, and is, for example, 5 to 6 seconds.

The method including contact with plasma gas is performed by generating plasma of a process gas containing nitrogen gas or argon gas as a main component and containing 0.01 to 5 vol% of oxygen gas by atmospheric-pressure plasma and bringing the plasma into contact with the principal surface 20a of the second substrate 20 and the principal surface 10a of the first substrate 10. It is also possible to use a mixed gas of nitrogen gas and clean dry air (CDA). The time of contact with the plasma gas is, for example, 5 to 100 seconds.

Then, a bonding step is performed in which the first substrate 10 and the second substrate 20 are stacked so that the bonding surfaces (10a, 20a) of both of the substrates subjected to surface activation treatment are in contact with each other, and both of the substrates are bonded together by pressing using a press machine. The bonding step should be performed within a predetermined time, for example, within 10 minutes after the completion of the ultraviolet irradiation step in order to maintain the surface activation state.

If necessary, the bonding step is performed in a thermal environment to achieve tight bonding. In the bonding step, bonding conditions such as heating temperature and pressing force are set depending on the constituent material of the first substrate 10 and the constituent material of the second substrate 20. As for specific conditions, the temperature during pressing is, for example, 40 to 130°C, and the pressing force for bonding is, for example, 0.1 to 10 MPa.

If necessary, a substrate obtained by bonding the first substrate 10 and the second substrate 20 together (hereinafter sometimes referred to as a "bonded substrate") may further be heated for a predetermined time after pressurization for a predetermined time. Even when a portion achieving a satisfactory bonding state and a portion not achieving a satisfactory bonding state are mixed at a bonded interface between the stacked substrates after pressurization, a desired bonding state can be achieved in the portion not achieving a satisfactory bonding state by heating the bonded substrate after pressurization.

After the pressurization of the bonded substrate is maintained for a predetermined time and then stopped, the temperature of the bonded substrate may be increased to and maintained at a predetermined temperature until a desired bonding state is achieved. Here, the predetermined temperature is a temperature at which deformation of the bonded substrate does not occur. For example, a heating temperature is, for example, 40 to 130°C and a heating time is, for example, 60 to 600 seconds.

Then, the culture vessel 1 in which the second substrate 20 is bonded onto the principal surface 10a of the first substrate 10 is produced through a cooling step.

### [Method of use]

Hereinbelow, a method of using the culture vessel 1 will be described in detail.

### <Usage Example 1 >

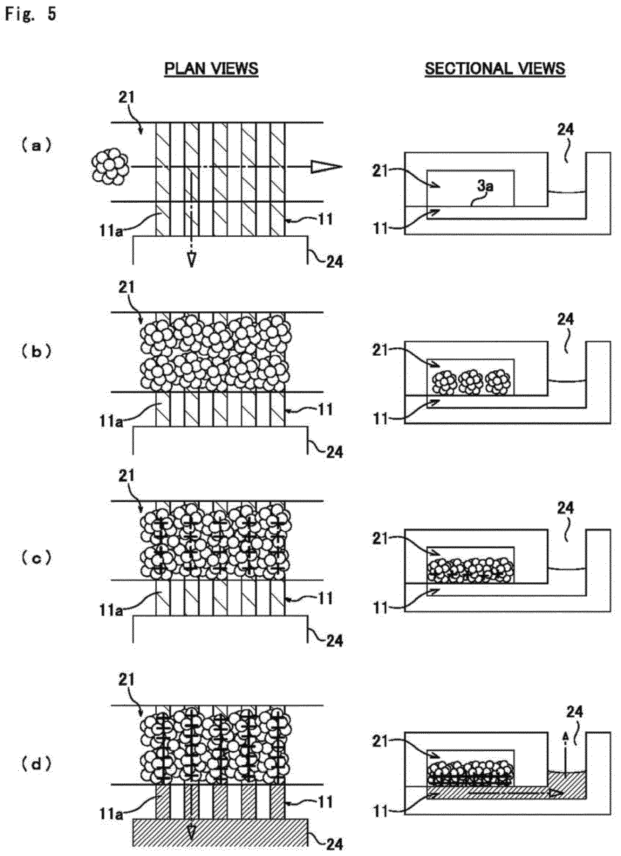

Fig. 5 is a schematic diagram for describing usage example 1 of the culture vessel 1, in which plan views are shown on the left side and sectional views are shown on the right side. It should be noted that in the plan views, the grooves 11a are indicated by hatched lines.

i) First, as shown in Fig. 5(a), hepatocytes are charged through the supply port (e.g., the first port 22) to be inoculated in the main flow path 21 (first step). At this time, the hepatocytes are charged as clusters of cells. The size of each of the clusters of cells is, for example, about 50 to 300 µm.

As shown in Fig. 5(b), the clusters of cells introduced into the main flow path 21 adhere to the bottom surface (contact surface 3a) of the main flow path 21 (second step). By setting the width 11W of each of the grooves 11a to be smaller than each of the clusters of cells, the clusters of cells adhere onto the secondary flow path 11 so as to cover the top of the grooves 11a.

ii) Then, as shown in Fig. 5(c), a culture solution is perfused by injecting it through the supply port and collecting it through the discharge port (e.g., the second port 23) to culture the hepatocytes (third step). The hepatocytes can be cultured in an environment similar to in vivo because the main flow path 21 has a geometrically small size.

The cells grow so that most or all of a space above the secondary flow path 11 is filled with the cells. As the hepatocytes grow, bile canaliculi (bile ducts) are formed in the hepatocytes.

iii) Then, a physiologically active substance or a drug of interest is charged through the supply port.

iv) Then, as shown in Fig. 5(d), fluid secreted from the bile canaliculi flows into the grooves 11a and is collected through the collection port (e.g., the third port 24) (fourth step). Since most or all of the space above the secondary flow path 11 is covered with the cells at this time, it is possible to almost completely or completely prevent the culture solution in the main flow path 21 from flowing into the secondary flow path 11. This makes it possible to collect fluid secreted from the cells at a high concentration.

This method makes it possible to collect bile excreted from the bile canaliculi constructed between the hepatocytes. Further, it is possible to evaluate the metabolism of a physiologically active substance or a drug in hepatocytes in an environment simulating an in vivo environment.

### < Usage Example 2 >

In the culture vessel 1, a perfusion system can be constructed by separately providing a pump, a culture medium reservoir, and an oxygen supply unit.

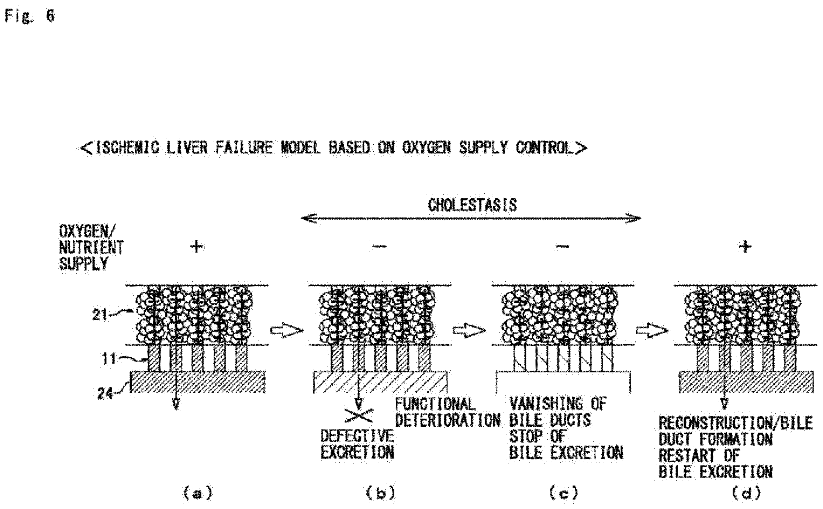

As shown in Fig. 6, ischemic hepatocyte damage, liver dysfunction caused thereby, and cholestasis caused by vanishing of bile ducts can be modeled by limiting the supply of oxygen and nutrients to a culture medium with the use of a perfusion system. Further, bile duct reconstruction and restart of bile excretion due to hepatic function recovery therefrom can be modeled. Fig. 6(a) shows a state where oxygen and nutrients are sufficiently supplied. Fig. 6(b) shows a state where the supply of oxygen and nutrients is insufficient so that liver function deteriorates, resulting in defective bile excretion. When insufficient supply of oxygen and nutrients continues, as shown in Fig. 6(c), bile ducts vanish so that bile excretion stops. When oxygen and nutrients are again sufficiently supplied, as shown in Fig. 6(d), bile ducts are reconstructed so that bile excretion restarts.

### <Usage Example 3>

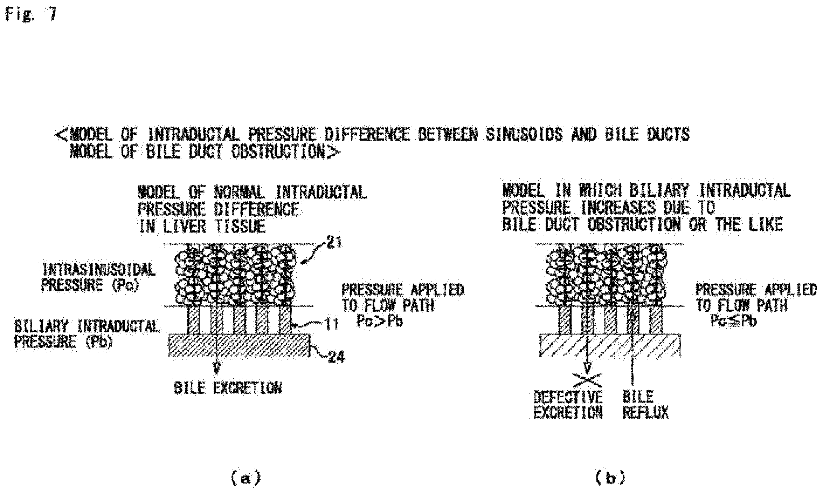

As shown in Fig. 7(a), bile excretion can be modeled by adjusting the inner pressure Pc of the main flow path 21 corresponding to hepatic blood vessels (sinusoids) and the inner pressure Pb of the secondary flow path 11 corresponding to bile ducts to the pressure in haptic blood vessels and the pressure in bile ducts in vivo, respectively so that a pressure difference similar to that in living tissue contributes to material transfer. Further, as shown in Fig. 7(b), an increase in the pressure in bile ducts caused by biliary tract obstruction or bile duct obstruction can be modeled by intentionally increasing the bile duct inner pressure Pb. When the pressure in bile ducts increases, defective bile excretion or bile reflux occurs.

### <Usage Example 4>

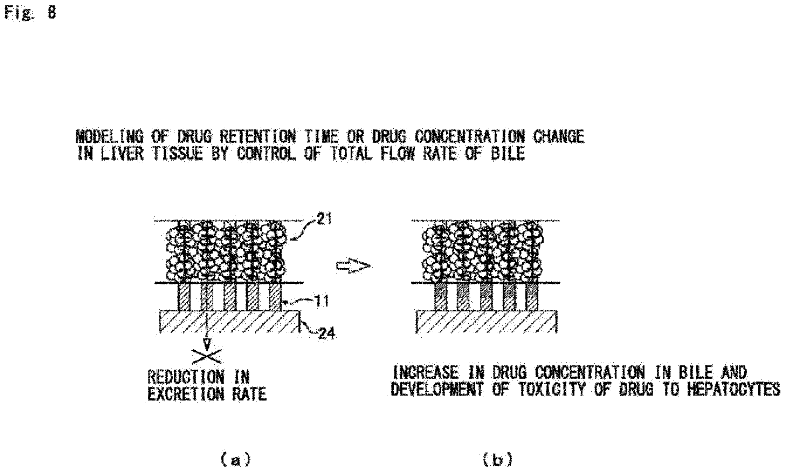

The state of defective bile excretion can be modeled by controlling the excretion rate of bile. In Fig. 8(a), the excretion rate of bile is reduced by, for example, increasing the pressure in the secondary flow path 11. By charging a drug into the main flow path 21 in a state where the amount of bile excreted is limited as shown in Fig. 8(a), excretion of the drug to the outside of hepatic tissue can be inhibited to intentionally increase the drug concentration in the hepatic tissue as shown in Fig. 8(b). This makes it possible to model the toxicity of the drug to hepatic tissue developed by bile duct obstruction or cholestasis.

### < Usage Example 5 >

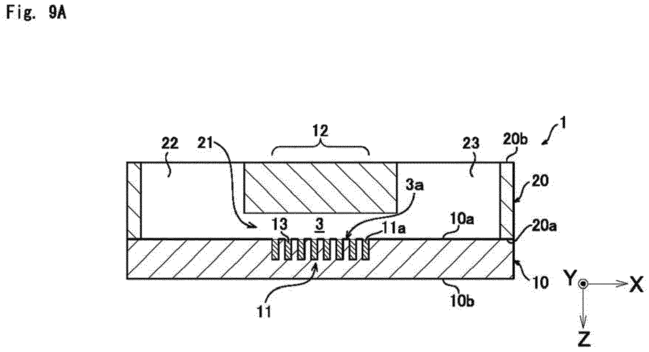

As shown in Fig. 9A, the culture vessel 1 may be configured so that the grooves 11a are filled with a gel 13. The gel 13 may be, for example, a collagen gel. A collagen gel is a gel constituted from collagen fibers. Filling the grooves 11a with the gel 13 makes it possible to culture cells in the culture space 3 even when the cells are smaller than the width 11W of each of the grooves 11a.

It should be noted that the gel 13 may be removed after the cells grow to a size such that they do not fall into the grooves 11a. The gel 13 can be removed by any appropriate method such as spontaneous decomposition, thermal dissolution, or enzymatic decomposition. Even after the gel 13 is removed, membranes or the like of the cells cultured in the culture space 3 are supported by the principal surface 10a between the adjacent grooves 11a, and therefore the form thereof is maintained.

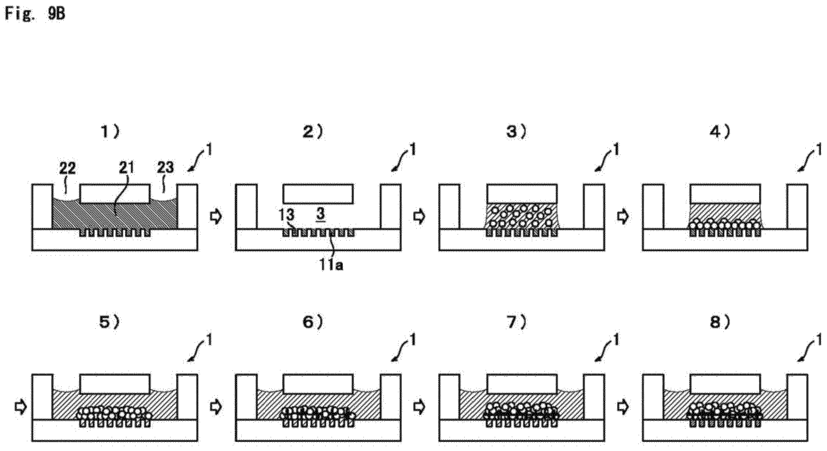

Fig. 9B shows sectional views for describing usage example 5 of the culture vessel 1. First, 1) the main flow path 21 is filled with a solution for forming a gel. Then, 2) only the solution for forming a gel filled in the grooves 11a is left and gelatinized. Then, 3) hepatocytes are inoculated. Then, 4) the hepatocytes are fixed to the culture space 3 (30 minutes to 1 hour). Specifically, the hepatocytes are allowed to adhere to the contact surface 3a as the bottom surface of the culture space 3. Then, 5) the first port 22 and the second port 23 as well as the culture space 3 are filled with a culture medium to grow the hepatocytes to obtain hepatic tissue (several days). Then, 6) bile canaliculi are formed in the hepatic tissue. Then, 7) the bile canaliculi open to the grooves 11a. Then, 8) bile is discharged into the grooves 11a.

### <Usage Example 6>

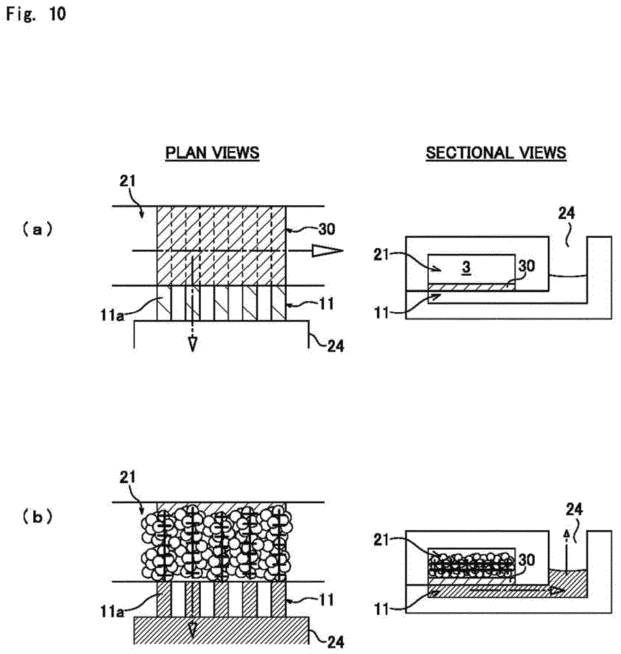

Fig. 10 shows plan views and sectional views for describing usage example 6 of the culture vessel 1. As shown in Fig. 10(a), cells may be cultured on a scaffold material 30 disposed on the upper surface of the secondary flow path 11. In this case, a space above the scaffold material 30 corresponds to the culture space 3. As shown in Fig. 10(b), bile excreted from the cells is discharged into the grooves 11a through the scaffold material 30.

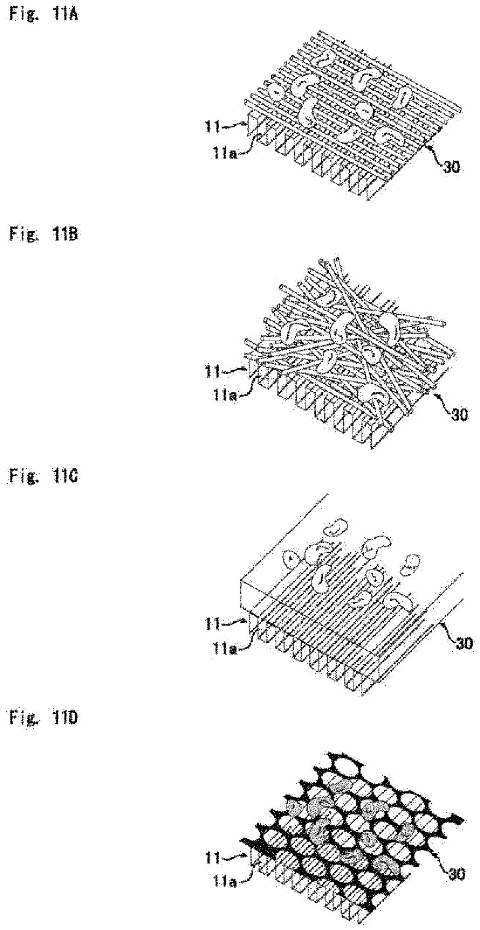

The scaffold material 30 may be fibers, a non-woven fabric, a mesh, or the like. Fig. 11A shows an example in which cells are cultured on silica or polymer fibers laid on the upper surface of the secondary flow path 11 at intervals such that the cells do not fall into the grooves 11a. In the example shown in Fig. 11A, the fibers are disposed as a single layer. Each of the fibers has a diameter of 100 nm to 1 µm and a length of about 1 mm to 100 mm. In this case, a water-soluble polymer or the like may be filled as a support in the grooves 11a of the secondary flow path 11 and then removed by dissolution after the fibers are disposed.

In the example shown in Fig. 11A, the fibers are disposed at almost regular intervals as a single layer. However, as shown in Fig. 11B, the fibers may overlap each other in the thickness direction so that cells do not fall into the grooves 11a. When the fiber layer is thick, it takes time for fluid secreted from cells, such as bile, to seep out. Therefore, the fiber layer is preferably thin, e.g., about 100 nm to 20 µm. Such a fiber layer can be formed by spraying a solvent in which fibers are dispersed onto the secondary flow path 11 and drying the fibers. In this case, the grooves 11a of the secondary flow path 11 may previously be filled with a gel, and then the gel may be removed after the fibers are sprayed and dried.

Fig. 11C shows an example in which cells are cultured on a non-woven fabric laid on the upper surface of the secondary flow path 11. The non-woven fabric may be formed using silica or a polymer. When the non-woven fabric is thick, it takes time for fluid secreted from cells, such as bile, to seep out. Therefore, the non-woven fabric is preferably thin, e.g., about 10 µm to 100 µm.

Fig. 11D shows an example in which a mesh made of silica or a polymer and having openings such that cells do not fall into the grooves 11a is laid on the upper surface of the secondary flow path 11, and cells are cultured on the mesh. Each of the openings of the mesh has a diameter of about 10 µm to 50 µm. When being polygonal, each of the openings have a size such that its corners are inscribed in a circle having a diameter of about 10 µm to 50 µm. The openings may be formed by patterning using lithographic exposure and then etching. A commercially-available mesh or membrane may also be used.

As described above, the method of using the culture vessel 1 according to the present embodiment may include: a first step of introducing cells into the main flow path 21; a second step of fixing the cells to the culture space 3; a third step of culturing the cells; and a fourth step of collecting fluid excreted from the cultured cells into the grooves 11a.

In the method of using the culture vessel 1 according to the present embodiment, the cells may be clusters of cells. In this case, the method may further include, before the first step, the step of preparing clusters of cells.

The use of clusters of cells makes it possible to increase the width 11W of each of the grooves 11a. As a result, it is possible to increase the amount of a solution collected from the secondary flow path 11 and the speed of collecting the solution.

In the method of using the culture vessel 1 according to the present embodiment, the second step may include the step of making the pressure in the grooves 11a lower than that in the main flow path 21 to adsorb the cells to the contact surface 3a.

Such a configuration makes it possible to gather cell adhesion areas at the contact surface 3a. As a result, it is possible to increase the proportion of cells whose secretion can be collected.

As described above with reference to the method of using the culture vessel 1 according to the present embodiment, the third step or the fourth step may include the step of maintaining the pressure Pb in the grooves 11a at a level lower than the pressure Pc in the main flow path 21.

This configuration makes it possible to prevent the cells from being removed from the contact surface 3a. Further, when hepatocytes are used as in the case of usage example 3, this configuration is the same as the internal environment of the body in that the pressure on the secretion collection side is maintained at a lower level, and is therefore effective also from the viewpoint of mimicking the internal environment of the body.

As described above with reference to the method of using the culture vessel 1 according to the present embodiment, the third step or the fourth step may include the step of maintaining the pressure Pb in the grooves 11a at a level equal to or higher than the pressure Pc in the main flow path 21.

This configuration makes it possible to reproduce the same state as cholestasis on the culture vessel as in the case of usage example 3.

Although the embodiments of the present invention have been described above with reference to the drawings, it should be understood that specific configurations are not limited to these embodiments. The scope of the present invention is indicated not only by the above description of the embodiments but also by the claims, and all changes which come within the meaning and range of equivalency of the claims are therefore intended to be embraced therein.

The structure adopted in each of the above embodiments can be adopted in any other embodiment. Specific configurations of parts are not limited only to those in the above-described embodiments, and various modifications can be made without departing from the spirit of the present invention.

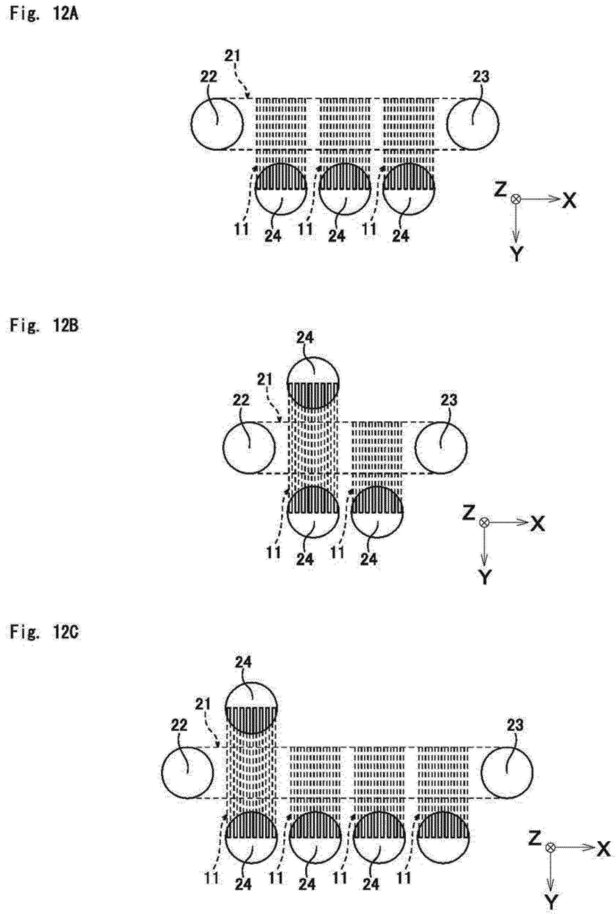

(1) In the above embodiments, only one collection port (third port 24) is provided. However, two or more collection ports may be provided. Fig. 12A shown an embodiment in which three third ports 24 are provided side by side. It should be noted that in Fig. 12A, the main flow path 21, the secondary flow paths 11, the first port 22, the second port 23, and the third ports 24 are schematically shown for the purpose of illustration (the same applies to Fig. 12B and Fig. 12C).

Further, Fig. 12B shows an embodiment in which the secondary flow paths 11 are provided so as to intersect the main flow path 21, and the third port 24 is provided at each of the ends of one of the secondary flow paths 11. Such a configuration makes it possible to introduce a liquid through one of the third ports 24 and collect the liquid through the other third port 24. Fig. 12C shows an embodiment achieved by combining the embodiment shown in Fig. 12A and the embodiment shown in Fig. 12B.

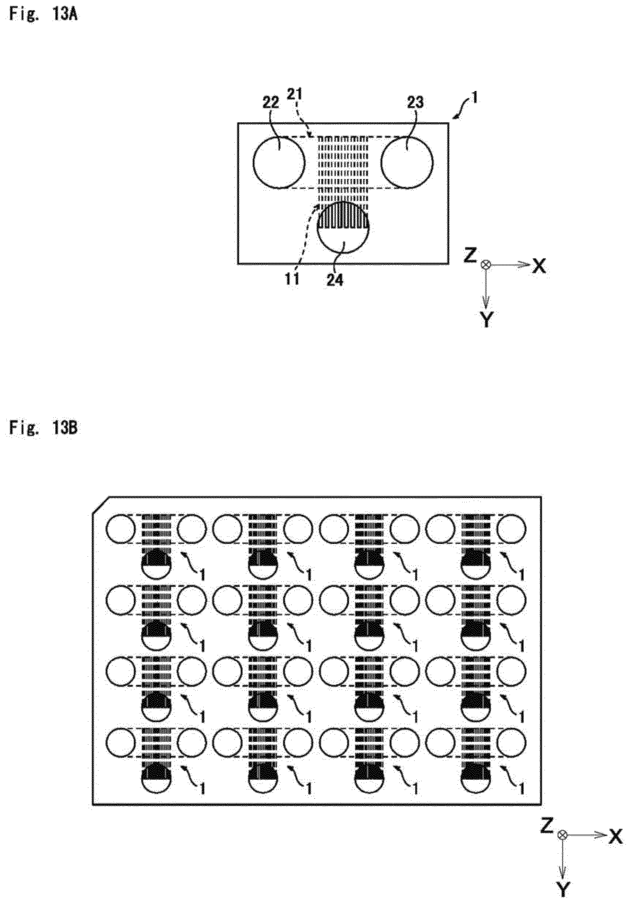

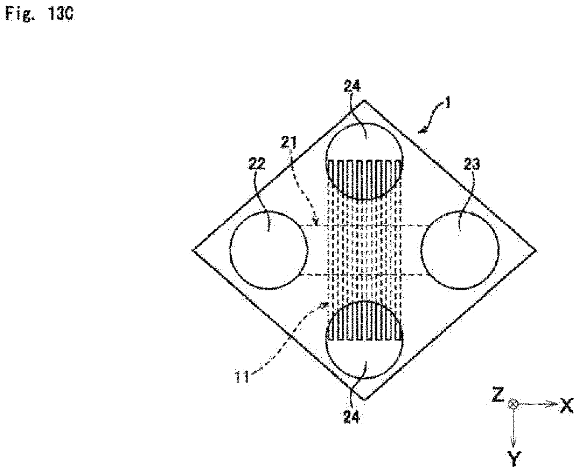

(2) In the above embodiments, the first substrate 10 and the second substrate 20 have an almost T-shape when viewed from the Z direction. However, the shape of the first substrate 10 and the second substrate 20 is not limited thereto. As shown in Fig. 13A, the first substrate 10 and the second substrate 20 may have a rectangular shape when viewed from the Z direction. As shown in Fig. 13B, two or more culture vessels 1 may be formed on one plate. As shown in Fig. 13C, the culture vessel 1 may have an almost diamond shape so that the third port 24 is provided at each of the ends of the secondary flow path 11. Such a configuration makes it possible to introduce a liquid through one of the third ports 24 and collect the liquid through the other third port 24.

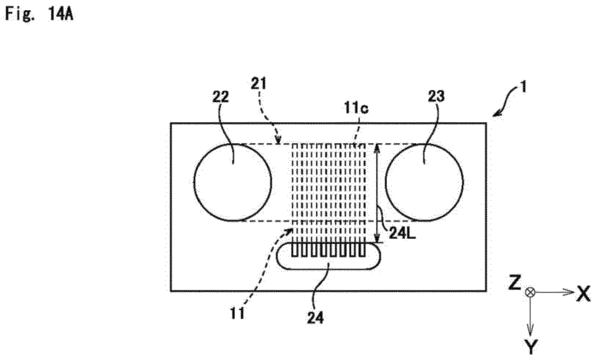

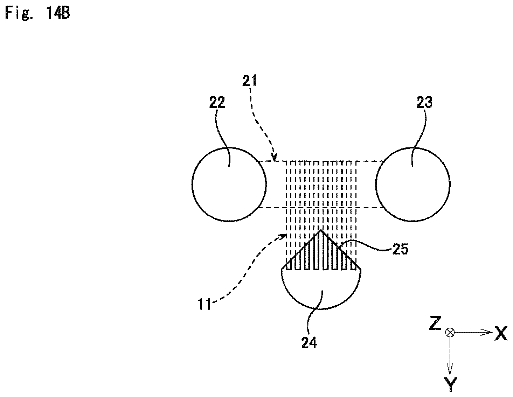

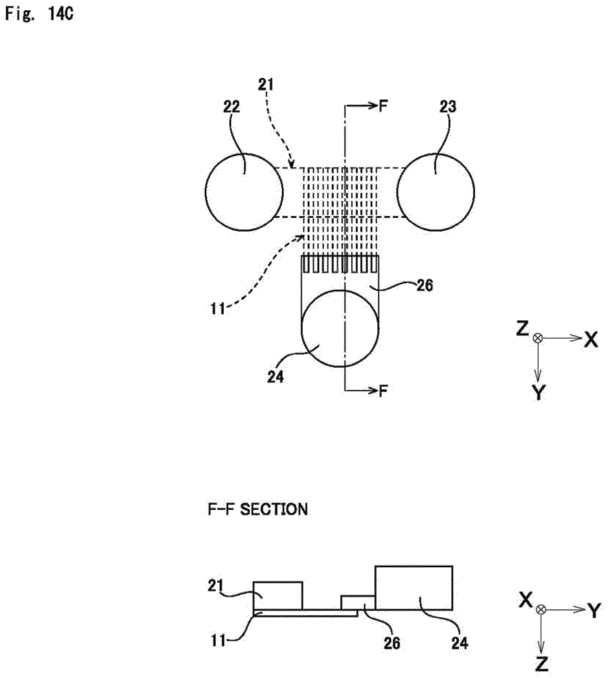

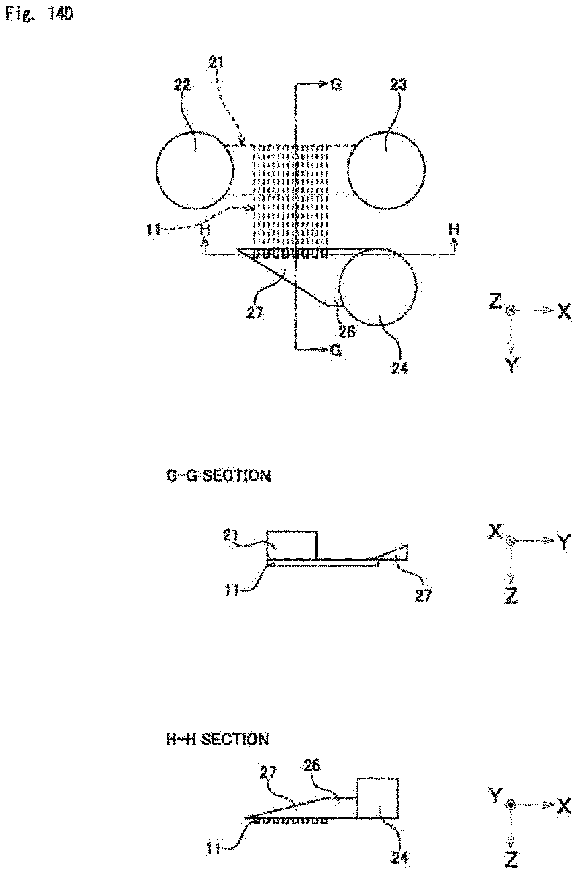

(3) In the above embodiments, the collection port (third port 24) has a circular sectional shape. However, the sectional shape of the collection port is not limited thereto. For example, as shown in Fig. 14A, the sectional shape of the third port 24 may be a rod shape whose longitudinal direction is parallel to the X direction. In the example shown in Fig. 13A, the lengths 11L themselves of the grooves 11a are constant, but distances from the other ends 11c as inlets of the grooves 11a to the edges of openings provided at the third port 24 as outlets of the grooves 11a are different, and therefore extraction time varies. On the other hand, in the case of the configuration shown in Fig. 14A, distances 24L from the other ends 11c as inlets of the grooves 11a to the edges of openings provided at the third port 24 as outlets of the grooves 11a are constant, and therefore extraction time does not vary. Fig. 14B shows a configuration in which part of the third port 24 forms an acute-angled area 25 that protrudes toward the secondary flow path 11 connected thereto. The acute-angled area 25 makes it possible to efficiently collect a small amount of fluid collected from cultured cells due to capillary action. Further, Fig. 14C shows a configuration in which a collection flow path 26 is provided on the outlet side of the grooves 11a constituting the secondary flow path 11, and this collection flow path 26 is connected to the third port 24. As shown in Fig. 14D, this collection flow path 26 may further have a squeezing area 27 in which opposed flow path walls constituting the flow path are close to each other. This makes it possible to efficiently collect a small amount of fluid extracted from cells due to capillary action that occurs in the squeezing area 27.

(4) In the culture vessel 1 shown in Fig. 12A, the secondary flow paths 11 (grooves 11a) connected to the respective collection ports (third ports 24) are the same in length. However, the lengths of the secondary flow paths 11 are not limited thereto. The secondary flow paths 11 connected to the respective collection ports may be different in length.

(5) The grooves 11a constituting the secondary flow path 11 do not have to be arranged in parallel, and may be formed in a matrix. Such a configuration makes it possible, even when any of the grooves 11a is obstructed, to bypass the obstructed groove 11a.

(6) In the above embodiments, hepatocytes (hepatic cells) are exemplified as cells to be cultured. Parenchymal cells can be used as cells that form clusters. Interstitial cells can be used as cells that form membranes (layers).

Cells that form clusters are parenchymal cells, and examples thereof include hepatic parenchymal cells and pancreatic islet cells.

Cells that form membranes are interstitial cells, and examples thereof include epithelial and endothelial cells such as alveolar epithelial cells, tracheal/bronchial epithelial cells, gastrointestinal/intestinal epithelial cells, biliary epithelial cells, breast ductal epithelial cells, epidermal cells, mucosal epithelial cells, nasal cavity/pharynx epithelial cells, renal tubular epithelial cells, urothelial cells, corneal epithelial cells, retinal tissue cells, exocervical epithelial cells, fibroblast cells, and cancer cells thereof. Other examples include vascular endothelial cells and lymphatic endothelial cells.

When cells that form membranes are inoculated, clusters formed from the cells may be used.

Further, stem cells such as embryonic stem cells, adult stem cells, or iPS cells introduced into the culture area may be differentiated into such cells as described above. Such differentiated cells may also be used as cells to be cultured.

### DESCRIPTION OF REFERENCE SIGNS

- 1: Culture vessel
- 3: Culture space
- 3a: Contact surface
- 11: Secondary flow path
- 11a: Groove
- 12: Overlapping area
- 21: Main flow path
- 21a: Recess
- 22: First port
- 23: Second port
- 24: Third port
- 25: Acute-angled area
- 26: Collection flow path
- 27: Squeezing area

## Claims

1. A culture vessel that has a culture space therein and is capable of culturing cells in the culture space, the culture vessel comprising:
a main flow path that extends within a plane; and
a secondary flow path that is adjacent to the main flow path in a direction perpendicular to the plane, extends in a direction intersecting the main flow path when viewed from a direction perpendicular to the plane, and partially overlaps the main flow path,
wherein the secondary flow path has a groove that is open to and communicates with the main flow path in an area where the secondary flow path overlaps the main flow path, and
the culture space is a space within the main flow path, the space being opposed to a contact surface where the secondary flow path is in contact with the main flow path in the area where the secondary flow path overlaps the main flow path.

2. The culture vessel according to claim 1, wherein the contact surface has cell adhesiveness.

3. The culture vessel according to claim 1 or 2, wherein a width of the main flow path is larger than each of the cells to be cultured, and a width of the groove is smaller than each of the cells to be cultured.

4. The culture vessel according to any one of claims 1 to 3, wherein the main flow path has a width of 1 to 10 mm, and the groove has a width of 5 to 100 µm.

5. The culture vessel according to any one of claims 1 to 4, wherein the groove comprises a plurality of grooves provided side by side and having a constant length.

6. The culture vessel according to any one of claims 1 to 5, wherein the groove is filled with a gel.

7. The culture vessel according to claim 1, comprising a collection port that communicates with the secondary flow path, wherein an acute-angled area is provided in a part of a wall surface of the collection port.

8. The culture vessel according to claim 1, comprising a collection flow path that communicates with the secondary flow path.

9. The culture vessel according to claim 8, wherein the collection flow path has a squeezing area in which flow path walls forming the flow path are close to each other.

10. The culture vessel according to any one of claims 1 to 9, wherein the cells to be cultured are of at least one type selected from among hepatic cells, pancreatic cells, spleen cells, kidney cells, intestinal epithelial cells, and vascular endothelial cells.

11. A method of using the culture vessel according to claim 1, the method comprising:
a first step of introducing cells into the main flow path;
a second step of fixing the cells to the culture space;
a third step of culturing the cells; and
a fourth step of collecting fluid excreted from the cultured cells into the groove.

12. The method of using the culture vessel according to claim 11, further comprising, when the cells are clusters of cells, a step of preparing the clusters of cells before the first step.

13. The method of using the culture vessel according to claim 11 or 12, wherein the second step comprises a step of making a pressure in the groove lower than a pressure in the main flow path to adsorb the cells to the contact surface.

14. The method of using the culture vessel according to any one of claims 11 to 13, wherein the third step or the fourth step comprises a step of maintaining a pressure in the groove at a level lower than a pressure in the main flow path.

15. The method of using the culture vessel according to any one of claims 11 to 13, wherein the third step or the fourth step comprises a step of maintaining a pressure in the groove at a level equal to or higher than a pressure in the main flow path.

16. The method of using the culture vessel according to any one of claims 11 to 13, comprising, before the first step, a step of filling the groove with a solution for forming a gel and turning the solution into a gel.
